# EUROPEAN PATENT APPLICATION

(11) **EP 3 483 253 A1**
(43) Date of publication of application: **15.05.2019**
(21) Application number: 17382749.4
(22) Date of filing: 08.11.2017
(51) Int. Cl.: C12M 1/00, C12M 3/00, C12M 1/12, A61L 27/24

(54) **CELLULARIZED COLLAGEN MEMBRANES INTO CELL CULTURE BAG**

(71) Applicant: Viscofan, S.A., 31192 Tajonar (Navarra) (ES); FIMA Fundación para la Investigación Médica Aplicada, 31008 Pamplona (Navarra) (ES)
(72) Inventor: ANDREU OLTRA, Enrique, José, 31008 Pamplona (Navarra) (ES); KOBLIZEK, Thomas, 40723 HILDEN (DE); PROSPER, Felipe, 31190 CIZUR MENOR (Navarra) (ES); HUSS, Annika, 67227 FRANKENTHAL (DE); QUINTANA, Lluís, 64646 HEPPENHEIM (DE)
(74) Representative: Herrero & Asociados, S.L.

(57) **Abstract**

The present invention refers to a porous plastic culture cell bag comprising a culture chamber delimited by the inner walls of the bag and at least two ports in one lateral side of the bag, wherein a collagen membrane is immobilized in one of the inner walls of the bag. The invention also refers to the method of manufacture this culture cell bag. Further, the method for cultivating cells in said cell culture bag is also contemplated. Finally, the invention also relates to a kit comprising a cellularized collagen membrane immobilized in one of the inner walls of the cell culture bag, and the method for manufacturing said kit.

## Description

### Field of the invention

The present invention refers to cultivation of cells in bags, in particular, porous plastic bags with a collagen membrane immobilized in one of its inner walls. The invention also relates to the manufacturing procedure of these bags and the method of cultivating the cells in said bags.

### Background of the invention

Regenerative medicine is the promised paradigm of replacement and repair of damaged or senescent tissues. As the building blocks for organ development and tissue repair, stem cells have unique and wide-ranging capabilities, thus delineating their potential application to regenerative medicine.

The present invention provides with a culture system which facilitates the growth of stem cells and its application in tissue regeneration.

The bio-processing industry has traditionally used stainless steel systems and piping in manufacturing processes for fermentation and cell culture. These devices are designed to be steam sterilized and reused. Cleaning and sterilization are however costly labour-intensive operations. Moreover, the installed cost of these traditional systems with the requisite piping and utilities is often prohibitive. Furthermore, these systems are typically designed for a specific process, and cannot be easily reconfigured for new applications. These limitations have led to adoption of a new approach over the last ten years-that of using plastic, single-use disposable bags and tubing, to replace the usual stainless steel tanks.

In particular bioreactors, traditionally made of stainless steel, have been replaced in many applications by disposable bags which are rocked to provide the necessary aeration and mixing necessary for cell culture. The bags are designed to maintain a sterile environment during operation thereby minimizing the risk of contamination.

Commonly used bags are of the "pillow style" mainly because these can be manufactured at low cost by seaming together two flexible sheets of plastic. Three dimensional bags have also been described, where further sheets may be used to create wall structures.

However, many cell types do not grow well in free suspension inside these bags for which, in larger scale cultivations, they are normally grown on microcarriers, i.e. particles providing surfaces for the cells to grow on.

US20140287512 discloses a cell culture bag manufactured from a flexible material, comprising microcarriers and cells and which further comprises a filter material attached to a wall of the culture bag. The cell culture is carried out inside the bag through the introduction of the culture medium, microcarriers and cells. Cells are adhered to the microcarriers and grow adhered to them. The method developed in this document improves cell culture in perfusion cultures. This method can deliver adherent cells in a suspension form, apt for production of biopharmaceutical products or for injection in tissue engineering applications. Nevertheless, these cells adhered to microcarriers can't be fixed in a particular tissue defect. They would need to be injected and therefore face typical efficacy problems related to cell loss.

Another kind of supports for cell growth has been described in the state of the art. Henke et al. (Journal of Tissue Engineering and regenerative medicine, 2014(8):248-252*)* disclose the manufacture of cell culture supports from oligo polyethylene glycol fumarate hydrogels, which, after being synthetized, are packaged in sterilization bags and sterilized by gamma irradiation. The objective of the bag is only to keep sterility of the supports as the cell culture is carried out outside the bag.

DE19964113 discloses chambers for the culture of skin cells comprising inside bags made from membranes, which serve as support for cell growth and that subsequently are applied to the damaged tissue. Such membranes, which are semipermeable and allow the passage of culture medium, oxygen and carbon dioxide, but not the passage of microorganisms, are prepared from collagen or cellulose. This manual multi-layer system can be used for sterile culture of cells on a membrane but would have difficulties in ensuring sterility and stability of the cell-seeded product in long-distance shipments.

Supports developed so far for cell culture do not allow their direct application in the repair of damaged tissue, without any manipulation. For this reason, it is necessary to find a suitable cellular support that allows to carry out cellular growth in conditions of sterility, guaranteeing the stability of the cell-seeded tissue engineering product during long-distance shipments and facilitating the direct application of the cell culture on the damaged tissue.

In response to the needs of the state of the art, the authors of the present invention have developed a new culture system that comprises a porous plastic cell culture bag having a collagen membrane, immobilized on one of its inner walls, which serves as support for the cell culture. The membrane remains static in the bag, also achieving a perfect adhesion of the cells to the membrane.

The bags disclosed in the present invention advantageously allow, once the incubation is finished, to remove the membrane from the culture bag and apply it directly on the damaged tissue with no manipulation, in sterility conditions.

### Brief description of the drawings

**Figure 1****.-** Collagen membrane attached to the cell culture bag, thermally sealed and filled with PBS: (p) Ports, (m) Membrane; (b) Bag.
**Figure 2****.-** Graphic showing cell viability of cells seeded onto collagen membrane, and incubated with a conservation medium (Hypothermosol) at 4°C, at 0, 24, 48 and 72 h.
**Figure 3****.-** Cellularized collagen membrane immobilized inside the cell culture bag of the invention and stained with crystal violet staining. A, B: cell culture bag edges are cut to remove cellular patch prior to implantation. C, D: cellularized collagen membrane is removed easily.
**Figure 4** A) PBS adhered membranes, detached. (B) Transported Tisseel membranes ("B" and "C") upon arrival to destiny. (C) "Tisseel C" membrane, transported and stained with crystal violet. (D) "Tisseel B" membrane, before trypsinization, and (E) after trypsinization.

### Detailed description of the invention

In a first aspect, the present invention discloses a porous plastic cell culture bag comprising:
- a culture chamber delimited by the inner walls of the bag and
- at least two ports in one lateral side of the bag,
wherein a collagen membrane is immobilized in one of the inner walls of the bag (hereinafter, "bag of the invention").

The plastic material of the bag of the invention is gas permeable, but impermeable to viruses or microorganisms, allowing cell culture in sterile conditions inside the bag, and transparent for microscopy. In a preferred embodiment, said material consists of a polyolefin. Both sides of the inner surfaces are identical and not coated.

The term "port" as used herein means an opening in one side of the bag, adapted for transport of material into or out of the bag.

In a preferred embodiment, the at least two ports comprise a luer lock port, to transfer fluids, and a septum closed port, to inject air in the bag. When handling the bag, it is necessary to inject air through one of the ports before removing the medium or other fluids. That is so that the top wall does not touch the cells and can fail them or take off the membrane. Septum port is further used to enable taking out samples of culture media during the manufacturing process, to test different parameters such as sterility, mycoplasma, etc, but all operations are performed with the luer-lock port.

Ports are often equipped with tube fitting. In particular embodiments, bag is equipped with a PVC tubing having a female luer lock with a male luer lock cap. Female luer locks allow for fluid transfer via a transfer device with a male luer lock. The luer lock prevents accidental disconnection during fluid transfer.

The term "immobilized" as used in the present invention refers to the physical reversible adherence of the collagen membrane to the inner surface of the bag. Immobilization enables the performance of cell culture protocols and shipments while avoiding undesired movement of membrane and cells until the product has reached operating theaters and is ready for implantation.

In a preferred embodiment, the area of the bag is between 0,01% and 100% bigger than that of the collagen membrane to enable thermal closure of the bag after fixation of the membrane on the bag. In a more preferred embodiment, the area of the bag is between 5% and 25% bigger than that of the collagen membrane in order to avoid gelatinization of the collagen too close to thermal closures and, at the same time, having coverage of most of the cell culture bag with collagen membrane to avoid loss of cells.

In a preferred embodiment, the collagen membrane comprises the following parameters:

| | |
|---|---|
| Collagen (% weight) | 50 a 70 approx. |
| Amide Nitrogen (% weight) | 0.14 to 0.4 approx. |
| Glycerine (% weight) | 12 to 35 approx. |
| Fat (% weight) | 0 to 7 approx. |
| Sorbite (% weight) | 0 a 20 approx. |
| Ash (% weight) | 0.5 to 3 approx. |
| Water (% weight) | 2 to 18 approx. |
| pH | 5.5 to 8 approx. |
| Weight per unit area (g/m²) | 20 to 40 approx. |
| Tensile strength(N/mm²) | 5 to 25 approx. |

In a second aspect, the present invention refers to a method for the manufacture of the porous plastic bag for cell culture of the invention comprising:
a) Cutting three lateral sides of the bag, with the exception of the side with the at least two ports;
b) Separating the bag walls and keep them open;
c) Dispensing a pharmaceutically accepted biologic adhesive on one of the inner walls of the bag;
d) Attaching the dry collagen membrane on the inner wall of the bag coated with the biologic adhesive,
e) Closing the open bag wall onto membrane,
f) Draining out the excess of adhesive,
g) Letting the bag dry for ≥ 30 minutes,
h) Sealing the 3 sides of the culture bag, and
i) Sterilizing the bag.

Prior to step a), it could be necessary to fill the culture bag with air, since both inside walls of the bag are probably closely attached.

In a preferred embodiment, the biologic adhesive dispensed in c) is based on fibrin. More preferably, the biologic adhesive based on fibrin comprises two components:
i. a sealant protein solution and
ii. a thrombin solution.

In order for the membrane to be firmly attached to the inner wall of the bag, alternating drops of the two components of the adhesive (i and ii) are placed. In this way, while the surface is being prepared, the adhesive still does not react. When the membrane is placed on the adhesive, and pressed, it is when the two components of the adhesive (which are between the membrane and the bag) are mixed, react and bond efficiently the membrane. This allows the membrane to be bonded throughout its surface to be as stable and homogeneous as possible. After this step, the excess liquid leaving the edges of the membrane when pressed is removed manually, using a Pasteur pipette (step f).

In a particular embodiment, solution i. comprises fibrinogen and aprotinin and solution ii. comprises thrombin and calcium chloride. Therefore, in step c), solutions i. and ii. of the adhesive are dispensed in alternating drops.

Sealing step (h) is performed through any process that ensures tightness of the sealed bag. In a particular embodiment, sealing can be performed through thermal sealing of the plastic bag, in which, through thermal heating, plastic walls are fluidized and pressed together to obtain a tight closed system.

The sterilization of the bag in step i) can be achieved by exposure to radiation, e.g. gamma, alpha or electron beam radiation, or by autoclaving.

In a preferred embodiment, the sterilization is through ionizing radiation, more preferably, through gamma irradiation.

All the steps must be performed in sterile conditions into a laminar flow cabinet.

In a third aspect, the present invention refers to a method for cultivating cells (hereinafter, "culture method of the invention") comprising:
- Providing the porous plastic culture cell bag of the invention,
- Filling the bag with culture media to equilibrate the collagen membrane from 10 to 120 minutes, at room temperature, through one of the at least two ports,
- Resuspending cells in culture media,
- Draining out the culture media from the bag and fill it with resuspended cells, obtained in the previous step, through said port, and
- Incubating the bag during a time comprised between 6 and 48 hours, at 37°C, to attach the cells onto the collagen membrane inside the bag.

In a preferred embodiment, cells are selected from stem precursor cells, embryonic stem cells, induced pluripotent stem cells or primary cells. In a more preferred embodiment, cells are mesenchymal stem cells, particularly adipose derived stem cells (ADSCs).

In a fourth aspect, the invention refers to a kit comprising a cellularized collagen membrane immobilized in one of the inner walls of the cell culture bag of the invention (hereinafter, "kit of the invention").

Finally, the last aspect of the invention refers to a method for the manufacture of the kit of the invention comprising:
- Culturing cells in a porous plastic bag according to the culture method of the invention,
- Draining out culture media contained in the bag through one of the at least two ports,
- Washing the bag with a buffer solution through said port,
- Draining out the buffer solution from the bag through said port,
- Adding a conservation medium, for the transport of the kit, through said port,
- Sealing the at least two ports and
- Storing at a temperature between 4 and 8 °C.

The kit can be shipped and transported ensuring the perfect adhesion of the cells to the membrane and guaranteeing the membrane remains static in the bag. Once the incubation is finished, the kit of the invention advantageously allows to remove the cellularized membrane from the culture bag and apply it directly on the damaged tissue with no manipulation, in sterility conditions.

### EXAMPLE 1

### MATERIALS

- Tisseel Duo: Tisseel component (diluted 1/15 in physiological serum). Thrombin component (dil. 1/50 in physiological serum)
- Culture bag: MACS GMP Cell Differentiation Bag-500 ref. 170-076-402 (5 bags). Miltenyi Biotec.
- Bag sealer: Thimonnier (HPL450AS)
- Tube sealer: Baxter HEMATRON 3 Bench Sealer
- Collagen Membrane Viscofan (11x11 cm) (as defined in *"*Preparation and characterization of collagen-based ADSC-carrier sheets for cardiovascular application", Arana M et al., 2013, Acta Biomater, 9(4):6075-6083*; "*Epicardial delivery of collagen patches with adipose-derived stem cells in rat and minipig models of chronic myocardial infarction", Arana M et al., 2014, Biomaterials, 35(1):143-151*).*
- Culture dish 500 cm² (Corning, ref. 431110)
- Scalpel
- Sterile gloves
- Sterile surgical field
- 50 ml luer lock Syringes
- Sterile Pasteur pipettes
- Sterile forceps
- Human Adipose Derived Mesenchymal Stem Cells (ADSC)
- Culture Media: alphaMEM+10%FBS+1ng/ml bFGF+P/S
- PBS
- HypoThermosol (BioLife Solutions)

### PROCEDURES

All procedures were performed in sterile conditions into a laminar flow cabinet.

### Attachment of collagen membrane into cell culture bag:

First, a sterile culture cell dish (500 cm²) was opened on sterile surgical field. Then, a sterile culture bag was located on the culture dish base. The culture bag was filled with air (because both inner walls of the bag were closely attached) with 50 ml syringe.

Three lateral sides of the bag (with the exception of the side with ports) were cut with a sterile scalpel on the culture dish base. Consequently, bag walls were separated and were kept open. Two separate 1 ml sterile Pasteur pipettes were filled each one with one of the two solutions included in Tisseel and approx. 25-50 µl drops of both alternative components were dispensed on one of the inner walls of the plastic bag. Then, a dry collagen membrane was placed and pressed over the surface where alternate drops of both Tisseel components had been placed in order to mix both Tisseel components drops and let them coagulate. At the same time the membrane got hydration.

Once pressed, the open wall of the bag was closed onto the membrane and subsequently the bag was spin around and the opposite bag wall was opened, keeping the membrane attached to the first wall. The excess fluid was drained out with a Pasteur pipette. The bag was dried at the air flow of the cabinet for at least 30 min. Then, the 3 sides of the culture bag were sealed with the bag sealer (both sealer positions in "10"). The seal was fitted to 4-5 mm of the border of the membrane.

The bag thus obtained was stored at 4°C until it was sterilized by Gamma-irradiation.

### Cell seeding:

Throughout a luer-lock port, the bag was filled with 50 ml of Culture Media, equilibrating the membrane, for 30 minutes, at RT.

72 million of human ADSC, isolated from lipoaspirates from donors, were resuspended in culture media. Then, the culture media, previously added in the bag, was drained out and the bag was filled it with 50 ml of the resuspended cells.

Those air bubbles appeared in the bag during the process were removed carefully Finally, the cell culture bag, with the membrane and cells, was incubated 24 hours at 37°C into a CO₂ culture incubator.

### Final product packaging:

Culture media was drained out from the bag with a 50 ml syringe throughout the luer-lock port.

The bag was washed with PBS x3 through the luer-lock port. Later, PBS was drained through the same port.

Then, 50 ml of Hypothermosol was added and the luer-lock port was sealed with tube sealer.

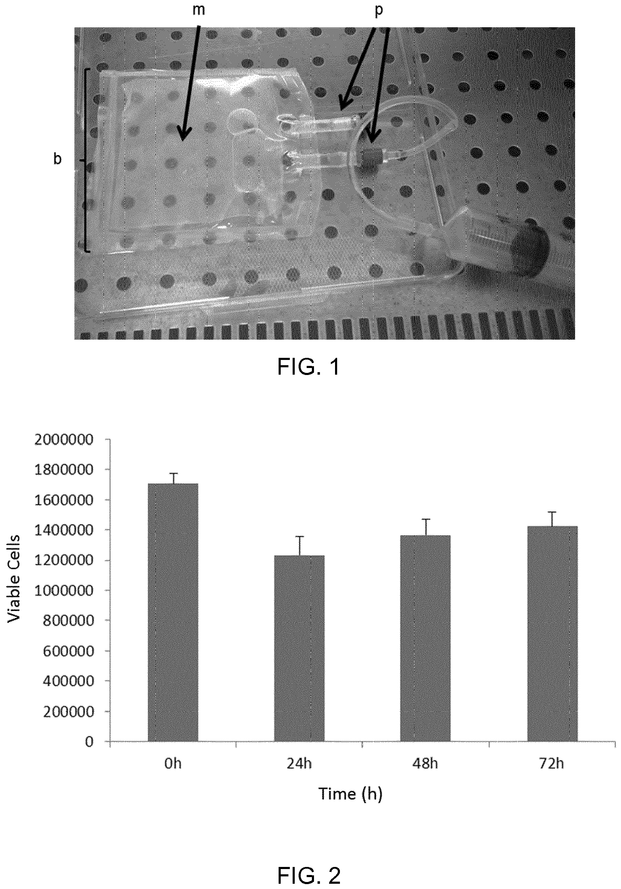

The final product thus obtained was stored at 4°C. Final product, i.e., the collagen membrane attached to the cell culture bag, thermally sealed and filled with PBS, is shown in figure 1.

### Cell viability assays:

Cell viability was assayed with CellTiter-Glo® Luminiscent Cell Viability Assay Kit (Promega). Results are shown in figure 2. Cell viability in this closed sterile system was given and stable until up to 72 h, enabling culture and shipment of cells on collagen membranes for periods up to 72 h without change of culture media. The initial reduction of cell viability observed at 24h was related to the number of cells that do not adhere to the membrane. Cells were seeded in excess and this excess was what explains the difference between viability at 0 and 24h.

### EXAMPLE 2

### MATERIAL

- 5 culture bags (Macs GMP Cell Differentiation bag-250. Miltenyi Biotec) with a collagen membrane immobilized: two adhered with PBS and three adhered with Tisseel (example 1).
- Tissue bag sealer: Thimonnier (HPL450AS)
- Plates for freezing bags in nitrogen
- 60-ml syringes
- Human ADSC (4 Hyperflasks expanded in CUN with cellbank 3P: hADSCs, batch 96304915003, frozen in cell passage 0)
- Culture medium DMEM+10% FBS+1 ng/ml bFGF+P/S
- PBS
- Hypothermosol
- Trypsin/EDTA (Lonza, BE17-161E)
- Crystal violet stain: 0.25 g crystal violet; 13.5 ml formaldehyde 37%; 50 ml PBS 10x; 5 ml methanol; 431.5 ml H2O.

### PROCEDURES

### Seeding of cells on the membranes:

50 ml of complete culture medium for membrane hydration was introduced through the luer-lock, equilibrating the membrane for at least 30 minutes. In order to distribute homogeneously and check bag tightness, the latter was placed in a bag freezing plate that was sealed with the cover under pressure. This allowed observation of any possible leak in the bag.

The ADSC of the 4 Hyperflasks were Trypsinized and detached.

Cells were counted and resuspended in 5 Falcon flasks containing 60 million each, and then 50 ml of culture medium was added.

The medium of the bag was discarded through the luer-lock with a syringe and the 50 ml of medium with the cells was injected through the lock (introducing a little air to ensure injection of the total volume).

All the air was eliminated from the bag with a syringe through the septum port, the bag was shaken to distribute the cells homogeneously, and placed in the freezing plate sealed under pressure to distribute the entire volume homogeneously over the surface of the membrane. Finally, the bag was placed in the incubator for 15 hours.

**Note:** the PBS bags were not processed, since the membranes would be detached during the process.

### Shipment of the cells

A 50-ml syringe was used to discard the culture medium from the three seeded bags.

Then, 50 ml of PBS was added and washed for at least 2 minutes, 3 times. Subsequently, 40 ml of Hypothermosol was added at 4°C.

Cold-stored bags were placed inside a zip bag in a carton box for shipping (Air Liquide), between two bubble bag laminas, to ensure homogeneous distribution of the Hypothermosol, and shipping from Pamplona to Madrid (Spain) by courier in cold package (GTC 28 L and a thermometer. +2°+8°C) of two of the three Tisseel bags (#B Tisseel and #C Tisseel). One was to remain in the Lab refrigerator (#A Tisseel). Ship placed in horizontal position.

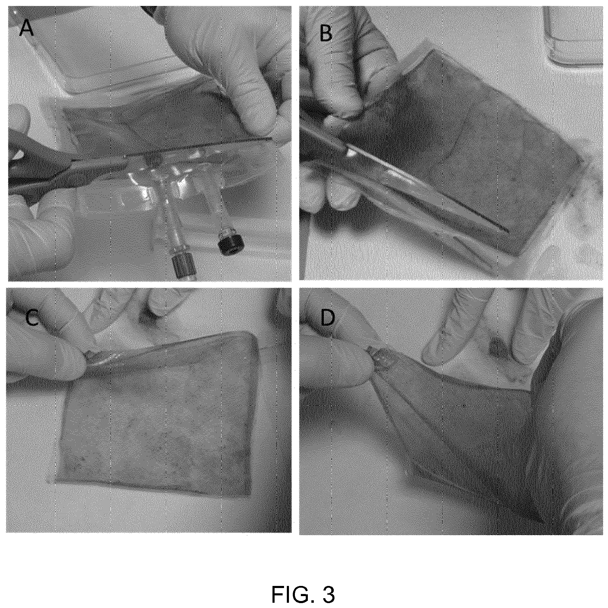

The package was changed in Madrid and returned back to Pamplona (Spain). There, checking was made of sterility, membrane adherence and cell adhesion and viability. Figure 3 shows how the membrane can be easily detached after a transport recovering a fully functional cell-seeded membrane.

### Sampling for sterility

In the experiments performed to validate this production process no contaminations were seen in any of the 7 batches produced and tested for sterility according to Eur. Ph. 2.6.1 (Steritest). Sterility was tested on the supernatant culture medium in which the cell-seeded membrane was cultured.

### Trypsinization for viability counting

Hypothermosol was fully extracted from the shipping bags (#B and #C) and from the bag kept in the laboratory (#A), with collection in different Falcon flasks. These were then centrifuged to obtain all the cells in the supernatant fraction. The cells were resuspended in approximately 500 µl and counted with viability testing:
#A**:** 1.28 million/ml in 400 µl = >500,000 cells in supernatant / 75% viability
**#B**: 1.96 million/ml in 750 µl = >1.47 million cells in supernatant / 61% viability
**#C**: 1.14 million/ml in 800 µl = >0.9 million cells in supernatant / 60% viability

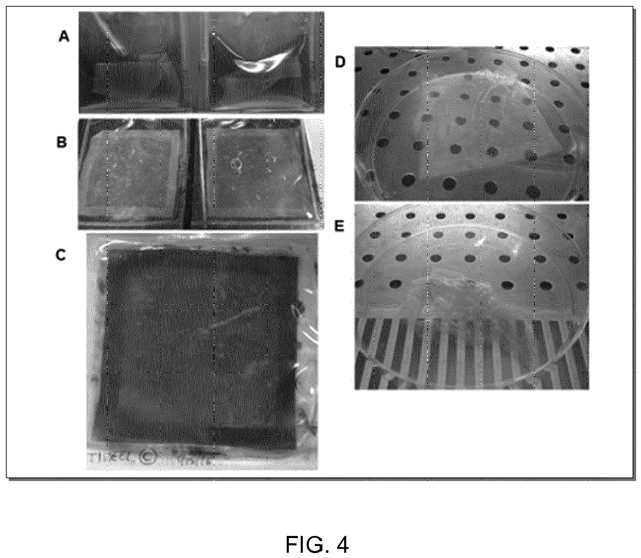

Bags #A and #B were opened (bag #C was destined for violet staining) and removed the membranes, placing them spread out on sterile Petri plates for trypsinization only of the membrane (figure 4D).

8 ml of trypsin/EDTA warmed to 37°C was added onto the membranes, taking care to prevent it from floating.

Then, they were incubated for 20 minutes at 37°C, shaking occasionally to confirm detachment.

8 ml of culture medium was added to inactivate, then shaked and collected all in a Falcon flask.

The membrane was washed twice with 8 ml of medium and collected each washing in a Falcon flask (end volume 30-35 ml).

Viability count with trypan blue was the following:
**#A**: 0.94 million/ml in approx. 30 ml = >28.2 million recovered cells / 100% viability
**#B**: 0.82 million/ml in approx. 32.5 ml = >26.7 million recovered cells / 100% viability

As can be seen in figure 4E, membrane was folded after trypsinization and thus it was difficult to ensure with this trypsinization in a Petri Dish, that all cells were recovered. This means that theoretically cell numbers could be higher than the ones shown in the experiment. In summary, results shown above were favorable and a good approximation to cell number on the membrane was obtained.

### Determination of Mesenchymal Phenotype:

Trypsinized cells were collected for phenotyping.

### Staining to check seeding homogeneity

Bag #C was stained (shipping). Then, 30 ml of crystal violet was added. Stain was placed at room temperature for 15 minutes. Then, bag was washed 3 times with PBS and left with 50 ml of PBS.

### RESULTS

The Tisseel membranes remained adhered without problems to the bags. The PBS adhered membranes did not resist the rehydration and seeding process and became detached (Figure 4A).

The cells distributed homogeneously on placing the bag in a freezing plate.

Shipment was correct. The temperature remained stable at 5.3-5.4°C. Packaging was perfect, since inside was a box containing the boxes with the bags in horizontal position in the bottom.

The shipped membranes (#B and #C) arrived fully adhered (Figure 4B); that retained in the laboratory (#A) became partially detached only after 9 months of storage.

Practically all the cells remained adhered, since the supernatant only yielded 0.9 and 1.5 million cells in the shipping bags, versus 0.5 million in the bag kept in the laboratory. On trypsinization of the bags, recovery in the shipping bag was practically the same as in the bag kept in the laboratory under resting conditions. Shipment did not appear to result in excessive detachment.

Staining of bag #C was perfect; the cellular zones (predominant) were clearly distinguishable from the non-cellular zones. Small denuded zones were seen where the cells had detached (Figure 4C).

The mesenchymal phenotype was maintained despite shipment in the bag. This was to be expected, since in such a short time and in the absence of factors, the cells are unable to differentiate.

## Claims

1. A porous plastic cell culture bag comprising:
- a culture chamber delimited by the inner walls of the bag and
- at least two ports in one lateral side of the bag,
wherein a collagen membrane is immobilized in one of the inner walls of the bag.

2. A porous plastic cell culture bag, according to claim 1, wherein the plastic material is gas permeable and transparent.

3. A porous plastic cell culture bag, according to claims 1 or 2, wherein the plastic material consists of a polyolefin.

4. A porous plastic cell culture bag, according to any of previous claims, wherein the area of the bag is between 0,01% and 100% bigger than that of the collagen membrane.

5. A porous plastic cell culture bag, according to claim 4, wherein the area of the bag is between 5% and 25% bigger than that of the collagen membrane.

6. A porous plastic cell culture bag according to any of claims 1-5, wherein the collagen membrane comprises the following parameters:
| | |
|---|---|
| Collagen (% weight) | 50 a 70 approx. |
| Amide Nitrogen (% weight) | 0.14 to 0.4 approx. |
| Glycerine (% weight) | 12 to 35 approx. |
| Fat (% weight) | 0 to 7 approx. |
| Sorbite (% weight) | 0 a 20 approx. |
| Ash (% weight) | 0.5 to 3 approx. |
| Water (% weight) | 2 to 18 approx. |
| pH | 5.5 to 8 approx. |
| Weight per unit area (g/m²) | 20 to 40 approx. |
| Tensile strength(N/mm²) | 5 to 25 approx. |

7. A porous plastic cell culture bag, according to any of claims 1-6 wherein the at least two ports comprises a luer lock port to transfer fluids and a septum closed port to inject air in the bag.

8. A method for the manufacture of a porous plastic cell culture bag, according to claim 1, comprising:
a) Cutting three lateral sides of the bag, with the exception of the side with the at least two ports;
b) Separating the bag walls and keep them open;
c) Dispensing a pharmaceutically accepted biologic adhesive on one of the inner walls of the bag;
d) Attaching a dry collagen membrane on the inner wall of the bag coated with the biologic adhesive,
e) Closing the open bag wall onto membrane,
f) Draining out the excess of adhesive,
g) Letting the bag dry for ≥ 30 minutes,
h) Sealing the 3 sides of the culture bag, and
i) Sterilizing the bag.

9. A method according to claim 8, wherein the biologic adhesive dispensed in c) is based on fibrin.

10. A method according to claim 9, wherein the biologic adhesive based on fibrin comprises two components:
i. a sealant protein solution and
ii. a thrombin solution.

11. A method according to claim 10 wherein the solution i. comprises fibrinogen and aprotinin and solution ii. comprises thrombin and calcium chloride.

12. A method according to claim 10 or 11 wherein, in step c), solutions i. and ii. of the adhesive are dispensed in alternating drops.

13. A method according to claim 8 wherein, in step i), the sterilization is through ionizing radiation.

14. A method, according to claim 13, wherein the ionizing radiation is gamma irradiation.

15. A method according to any of claims 8-14 wherein all the steps are performed in sterile conditions into a laminar flow cabinet.

16. A method for cultivating cells comprising:
- Providing a porous plastic cell culture bag according to any of claims 1-7,
- Filling the bag with culture media to equilibrate the collagen membrane from 10 to 120 minutes, at room temperature, through one of the at least two ports,
- Resuspending cells in culture media,
- Draining out the culture media from the bag and fill it with resuspended cells through said port, and
- Incubating the bag during a time comprised between 6 and 48 hours, at 37°C, to attach the cells onto the collagen membrane inside the bag.

17. Method according to claim 16 wherein cells are selected from stem precursor cells, embryonic stem cells, induced pluripotent stem cells or primary cells.

18. Method according to claim 17 wherein cells are mesenchymal stem cells.

19. Method according to claim 18, wherein cells are adipose derived stem cells (ADSCs).

20. A kit comprising a cellularized collagen membrane immobilized in one of the inner walls of a porous plastic cell culture bag according to any of claims 1-7.

21. Method for the manufacture of the kit, according to claim 20, comprising:
- Culturing cells in a porous plastic bag according to the method of claim 16,
- Draining out culture media contained in the bag through one of the at least two ports,
- Washing the bag with a buffer solution through said port,
- Draining out the buffer solution from the bag through said port,
- Adding a conservation medium for the transport of the kit through said port,
- Sealing the at least two ports and
- Storing at a temperature between 4 and 8 °C.
